# EUROPEAN PATENT APPLICATION

(11) **EP 3 006 936 A1**
(43) Date of publication of application: **13.04.2016**
(21) Application number: 14808440.3
(22) Date of filing: 03.06.2014
(51) Int. Cl.: G01N 31/00, G01N 31/22, G01N 33/24

(54) **SOIL MOISTURE INDICATOR**

(30) Priority: 06.06.2013 JP 2013003209; 23.07.2013 JP 2013152916
(71) Applicant: Cabinotier Co.,ltd., Tokyo 108-0073 (JP)
(72) Inventor: ORIHARA Ryo, Tokyo 108-0073 (JP)
(74) Representative: Müller-Boré & Partner Patentanwälte PartG mbB
(86) International application number: PCT/JP2014/064716
(87) International publication number: WO 2014/196521

(57) **Abstract**

To make it possible to visualize the drying of soil to a desired degree and notify a user of the drying. A soil moisture indicator (1) according to the present invention includes a main body (2) that is formed in a hollow shape from a material through which water does not pass and has a water absorption opening (5) disposed near one end in a longitudinal direction, an evaporation opening (7) disposed near the other end, and a display section (6) provided near the other end and formed to visualize the hollow inside; a water absorption material (3) that is filled on the inside of the main body (2) at least from the water absorption opening to the display section; and a water detection sheet (4) that is disposed to cover the water absorption material (3) at a position of the display section (6) inside the main body (2) and has varying color tones between a water absorption state and a dry state. The main body may be provided with a guide line (8) that indicates a target depth to which the main body can be inserted into the soil.

## Description

### Technical Field

The present invention relates to a soil moisture indicator which indicates a watering timing to maintain a moisture content of soil suitable for growth of plant.

### Background Art

An indicator that displays the moisture content to maintain the moisture content of the soil suitable for growth of the plant has been suggested. For example, a plant electronic soil humidity measuring device configured to measure the humidity of the soil based on a change in electrical resistance of a soil humidity sensing unit (see, for example, Patent Document 1), and an indicator which is provided with a porous layer having different transparency between a liquid absorption state and a non-liquid absorption state to come into contact with a water absorption material having a water absorption force, and makes colors different depending on the moisture content in the soil (see for example, Patent Document 2) have been suggested. Although a measurer called a pF meter are commercially available in addition to these indicators, similarly to the indicator illustrated in Patent Document 1, since it is necessary to read the indicated numerical values and the price is also high, it is not easily available.

Soil wetness is represented by a value called "pF value" . The pF value is a unit of a pressure which represents the degree of intensity by which water in the soil is drawn by a capillary force of the soil. In the case of the soil that sufficiently contains water, the pF value becomes smaller, which represents that the roots of the plant easily suck water. In contrast, when the soil dries, the pF value becomes higher, and high force is required to suck water. In the case of a farmland, the pF value is usually 1.5 to 2.7 ( growth available water), the moisture is excessive in the value below the range, and the moisture lacks in the value above the range.

Although there are differences in varieties of plants to be grown, the pF value in which plant does not have a stress is in the range of pF 1.7 to 2. 3. Therefore, the indicator needs to indicate that the soil excessively dries when exceeding the appropriate pF value depending on the plant. However, in the device described in Patent Document 1, a battery is required in the plant electronic soil humidity measuring device due to the use of electrical means for measuring the moisture content, and when the battery is exhausted, the measurement cannot be performed, and the display is not possible. Furthermore, since the moisture is not known unless the digital display is read, it is not possible to easily know the dryness of soil and the necessity of watering. Further, in the indicator of Patent Document2, although the battery is not necessary, it is difficult to adjust the moisture content (pF value) in which color is changed to an appropriate value depending on the variety of plants to be grown.

### Prior Art References

### Patent Document

Patent Document 1: JP 3070450 U
Patent Document 2: JP 3136622 U

### Summary of the Invention

### Problems to be solved by the Invention

The problem to be solved is to make it possible to visualize drying of the soil to the desired degree and to notify a user of the drying.

### Means for solving the Problems

The present invention solves the problem by the following configurations. Specifically, A soil moisture indicator according to the present invention includes a main body which is formed in a hollow shape by a material through which water does not pass, and has a water absorption opening disposed near one end in a longitudinal direction, an evaporation opening disposed near the other end, and a display section provided near the other end and formed to visualize the hollow inside, a water absorption material which is filled in the inside of the main body at least from the water absorption opening to the display section, and a water detection sheet which is disposed so as to cover the water absorption material at a position of the display section inside the main body and has varying color tones between a water absorption state and a dry state.

The water absorption material is preferably formed of a cotton cloth from which oil is removed. Thus, it is possible to enhance the water absorption force due to the capillary phenomenon, and it is possible to allow the water absorption material to serve as an indicator even in a long main body. To remove the oil of cotton, it is preferable to sufficiently perform a refining and bleaching process.

The evaporation opening is preferably formed on a side surface of the main body. Thus, it is possible to prevent the moisture from entering the inside of the main body from the evaporation opening when performing the watering.

At least a part of the evaporation opening is preferably provided on the other end side further than the display section. Accordingly, since the evaporating moisture passes through the display section, it is possible to stabilize the display using the indicator.

The water absorption opening is preferably formed on the side surface of the main body. It is possible to prevent the soil from being pressed to the inside of the main body when inserting the indicator into the soil, and it is possible to prevent the contact condition between the water absorption opening and the soil from changing depending on the insertion direction.

The main body is preferably formed of a resin material. Deterioration due to the action of moisture and microorganism in the soil is prevented, which makes it possible to repeatedly use the main body for a long period of time.

### Advantageous Effects of Invention

According to the soil moisture indicator of the present invention, it is possible to visualize the drying of the soil to a desired degree and easily notify the drying.

### Brief Description of the Drawings

Fig. 1 illustrates an external view of a soil moisture indicator 1.
Fig. 2 illustrates a cross-sectional view of the soil moisture indicator 1.

### Description of Embodiments

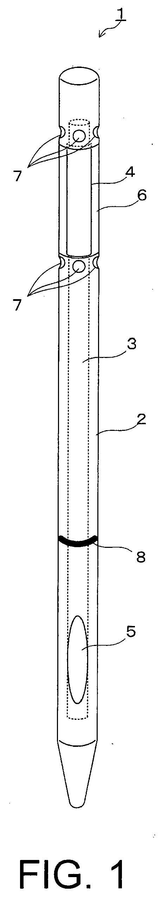

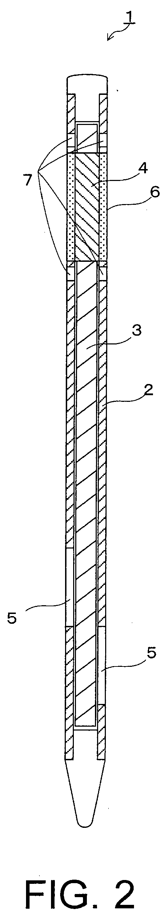

Hereinafter, an embodiment of the present invention will be described with reference to Figs. 1 and 2. Fig. 1 illustrates an external view of a soil moisture indicator 1, and Fig. 2 illustrates a cross-sectional view of the soil moisture indicator 1. As illustrated in Figs. 1 and 2, the soil moisture indicator 1 is equipped with a main body 2, a water absorption material 3, and a water detection sheet 4.

The main body 2 is formed in a hollow shape (for example, a cylindrical shape) by a material which does not allow water to pass therethrough. Specifically, although the main body 2 is made of any material as long as the material is a plastic such as an ABS resin or a metal which does not allow water to pass therethrough, a material which is not degraded under the action of moisture and microorganisms in the soil is preferable, and the ABS resin is preferable in this regard.

A water absorption opening 5 is provided on the side surface near the one end in the longitudinal direction of the main body 2. In this example, although the shape of the water absorption opening 5 has an elliptical shape, the shape of the opening is not limited thereto. The size of the opening is adjusted to a size by which the water absorption from the soil is sufficiently possible. A plurality of water absorption openings 5 may be provided. For example, as illustrated in Fig. 2, two openings may be provided at different positions in the longitudinal direction and the outer circumferential direction of the main body 2.

A display section 6 formed to visualize the inside of the hollow main body 2 is provided near the other end of the main body 2. Although the display section 6 is preferably formed of a transparent material, as long as a change in color tone of the water detection sheet 4 disposed in the main body 2 can be checked, the presence or absence of the color and transparency do not matter. Also, the portions except the display section 6 of the main body 2 may have any color or may be transparent. The display section 6 is preferably formed to visualize (for example, transparent) the inside over the entire circumference of the main body 2. Thus, it is possible to check the color tone of the water detection sheet 4 irrespective of the viewing angle.

The water absorption material 3 is filled in a portion at least from the water absorption opening 5 to the display section 6 in an internal space of the main body 2. The water absorption material 3 is sucked up moisture in the soil to a height of the display section 6 by the capillary phenomenon against gravity. In order to exert a sufficient suction force, a fine fiber is preferably used as the water absorption material 3. In addition, it is preferred that oil be sufficiently removed from the water absorption material 3. In addition, by performing the refining and bleaching process on cotton, a cotton wax as oil contained in the cotton can be removed. For example, as the water absorption material 3, it is preferable to use a material which has a rod shape by twisting the cotton cloth from which oil is removed by the refining and bleaching process. A configuration may be adopted which prevents the bacteria from being propagated on the inside of the water absorption material 3 in use, by immersing a preservative into the water absorption material 3 and drying it. When the water absorption force of the water absorption material 3 changes by immersing the preservative, the position and the size of the water absorption opening 5 and the evaporation opening 7 may be adjusted so that the change in color tone of the water detection sheet 4 occurs at a desired pF value by offsetting the change in water absorption force. On the side surface of the main body 2, it is preferable to provide a guide line 8 that serves as a measure of the depth to be inserted into the soil. The guide line 8 is provided at a position of about 5 cm in a direction of the end portion in which the evaporation opening 7 is provided from the water absorption opening 5, and the soil moisture indicator 1 is used in a state where the soil moisture indicator 1 is inserted to a depth at which the guide line is substantially coincident with the surface of the soil. In the use state, the water absorption material 3 exposed from the water absorption opening 5 sucks up water in the soil.

In a portion of the display section 6 in the main body 2, the water detection sheet 4 is disposed to cover the water absorption material 3. The water detection sheet 4 is a sheet-like material which contains a hydrochromic ink. The hydrochromic ink changes in color tone between the water absorption state and the dry state. That is, the water detection sheet 4 illustrates the different color tones when the moisture is sufficient in the soil and the water absorption material 3 sucks the moisture and is moist, and when the soil dries and the water absorption material 3 also dries. For example, it is preferable that the color tone clearly change between the water absorption state and the dry state, like the ink that illustrates the color tone such as white during drying, red and blue during water absorption. A change in the color tone can be observed from the display section 6. When the display section 6 is formed to visualize (for example, transparent) the inside over the entire circumference of the main body 2, the water detection sheet 4 preferably covers the visible range from the display section 6 of the water absorption material 3 over the entire circumference.

Although it is possible to use, for example, paper or cloth as a sheet-like material constituting the water detection sheet 4, the water detection sheet 4 is preferably a material having the water retention capability inferior to the water absorption material 3 so that the sheet-like material does not retain the moisture in the state in which the water absorption material 3 dries, and the water detection sheet 4 preferably has sufficient durability. Further, the water detection sheet 4 is preferably a material to which the hydrochromic ink is easily fixed. When using a cotton after removing the oil as the water absorption material 3, for example, as a sheet-like material constituting the water detection sheet 4, a nonwoven fabric, a cotton fabric in which the cotton wax remains greater than the water absorption material 3, a T / C broadcloth (broad fabric obtained by a blended yarn of cotton and polyester) or the like may be used.

The evaporation opening 7 is provided on the near side surface of the other end of the main body 2. The evaporation opening 7 may be provided only at the position of the other end side further than the display section 6, and may be provided only at the position of the one end (that is, an end portion in which the water absorption opening 5 is provided) side further than the display section 6. However, in this example, the evaporation opening 7 is provided on both sides of the display section 6. The evaporation opening 7 may be provided as a plurality of pores, and it is possible to set a pF value in which a change in color tone occurs by the number of pores. In the example illustrated in Fig. 1, although the shape of the evaporation opening 7 is circular, the shape of the opening is not limited thereto.

Ends of the both sides of the main body 2 are sealed by a material such as an ABS resin through which the moisture does not pass. By sealing the one end in which the water absorption opening 5 is provided, and by providing the water absorption opening 5 on the body side surface, it is possible to prevent the soil from being pressed into the inside of the main body 2 when inserting the one end into the soil, and it is possible to prevent the contact condition between the water absorption opening 5 and soil from changing depending on the insertion direction. Moreover, by sealing the end portion (the other end) of the side on which the evaporation opening 7 is provided, moisture can be prevented from entering the interior of the main body 1 from the end portion when performing the watering. The end portion of the side on which the water absorption opening 5 is provided is preferably formed in a shape with a pointed tip such as a conical shape. Insertion into the soil becomes easier by such a tip shape.

The main body 2 preferably has a full length of 5 to 40 cm so that the display section 6 is exposed from an edge of a container such as a flowerpot in the use state. Moreover, an outer diameter is preferably set to about 5 to 10 mm to facilitate the insertion into the soil, and an inner diameter is preferably set to about 4 to 8 mm to have sufficient strength.

The main body 2 may be produced by any method as long as it is finally formed as the aforementioned configuration, and the main body 2 may be formed, for example, by machining a tubular material and may be formed by a method of putting the water absorption material 3 to a member having a shape obtained by dividing the tubular member into half in the longitudinal direction, and then joining them.

The pF value by which the soil moisture indicator 1 indicates a change in color tone can be adjusted by various parameters. For example, it is possible to adjust the drying rate of the water absorption material 3 by the magnitude of the cross-sectional area of the evaporation opening 7, which makes it possible to adjust the pF value in which the change in color tone of the water detection sheet 4 occurs. Specifically, since the water absorption material 3 is more quickly dried if the cross-sectional area of the evaporation opening 7 increases, it is possible to adjust the pF value indicating the change in color tone to a small level (that is, so as not to react when moisture in the soil is not much). When the evaporation opening 7 is formed as a plurality of pores, it is preferable in that the pF value indicating the change in color tones can be adjusted by the number of pores.

It is possible to adjust the pF value indicating the change in color tone, depending on the length of the main body 2 (in particular, a length from the water absorption opening 5 to the display section 6), an inner diameter, a cross-sectional area of the water absorption opening 5 or the like, in addition to the evaporation opening 7.

The soil moisture indicator 1 is used, while inserting one end in which the water absorption opening 5 is provided into the soil. In the use state, the water absorption material 3 exposed from the water absorption opening 5 tries to suck up the moisture in the soil by the capillary phenomenon. When sufficient moisture exists in the soil and the pF value is low, the water absorption material 3 enters a wet state to the vicinity of the display section 6, and the water detection sheet 4 which covers the display section 6 becomes the color tone of the water absorption state in response to the moisture. Meanwhile, when the sufficient moisture does not exist in the soil and the pF value is high, the water absorption material 3 near the display section 6 enters a dried state, and the water detection sheet 4 which covers the display section 6 becomes the color tone of the dry state. With a change in color tone when the soil dries, the soil moisture indicator 1 can display the drying of the soil to a user. The soil moisture indicator 1 can be repeatedly used again and again, while changing between the water absorption state and the dry state, depending on the watering and the drying of the soil.

### Examples

### [Example 1]

The soil moisture indicator 1 was produced under the following conditions.

As the main body 2, a transparent ABS resin tube having an outline form of φ 6 mm, an inner diameter of φ 4 mm, a wall thickness of the tube of 1 mm and a full-length of 15 cm was used, a substantially elliptical water absorption opening 5 having a large diameter of approximately 15 mm and a short diameter of approximately 2 mm was provided by cutting the wall surface of one end of the tube, and in the other end of the tube, eight (total sixteen) pores (φ 2 mm) as the evaporation openings 7 were provided on each of both sides of the position which is the display section 6. Amaterial obtained by twisting the cotton cloth in a rod shape after performing the refining and bleaching process and removing the oil was prepared as the water absorption material 3, and the position viewed from the display section 6 when inserted into the tube of the main body 2 was covered with the water detection sheet 4. The water detection sheet 4 was previously produced, by applying a hydrochromic ink illustrating the color tones of red in the water absorption state and white in the dry state to a white T / C broadcloth fabric. The water absorption material 3 and the water detection sheet 4 were inserted into the tube of the main body 2 and both ends of the main body 2 were sealed.

The soil moisture indicator 1 was inserted into a flowerpot that grows Poulsen rose. At this time, the water absorption opening 5 was located at a depth of approximately 5 cm from the soil surface. Also, in order for the soil moisture indicator 1 to accurately measure the pF value which causes the change in color tone, a pF meter was inserted together. When performing the watering in the pot in this state, the water detection sheet 4 became red. Thereafter, the soil moisture indicator 1 was left without performing the watering, and the readings of the pF meter when the color tone of the water detection sheet 4 was changed to white were checked. When the watering and leaving of moisture were repeated, the pF value in which the color tone of the water detection sheet 4 changes to white was 2.1 to 2.2.

### [Example 2]

Except that the four (total eight) pores (φ 2 mm) as the evaporation openings 7 are placed on each of both sides of the position which is the display section 6, the soil moisture indicator 1 was produced in the same manner as in Example 1. When repeating the watering and leaving in the same manner as in Example 1, the pF value in which the color tone of the water detection sheet 4 changes to white was 2.6.

As is apparent from Examples 1 and 2, according to the soil moisture indicator of the present invention, it is possible to reproducibly visualize the moisture of the soil as a change in color tone of the water detection sheet 4. Also, for example, it is possible to adjust the pF value in which the change in color tone of the water detection sheet 4 occurs, by a structural parameter such as the cross-sectional area of the evaporation opening 7, and it is possible to produce an indicator which is suitable for a variety of plants.

### Industrial Applicability

Since the soil moisture indicator of the present invention can easily and visually check that the soil becomes a desired dryness, simply by being inserted into the soil of the flowerpot and the planter, it can be utilized for the growth of various plants.

### Reference Signs List

- 1: soil moisture indicator
- 2: main body
- 3: water absorption material
- 4: water detection sheet
- 5: water absorption opening
- 6: display section
- 7: evaporation opening
- 8: guide line

## Claims

1. A soil moisture indicator comprising:
a main body which is formed in a hollow shape by a material through which water does not pass, and has a water absorption opening disposed near one end in a longitudinal direction, an evaporation opening disposed near the other end, and a display section provided near the other end and formed to visualize the hollow inside;
a water absorption material which is filled in the inside of the main body at least from the water absorption opening to the display section; and
a water detection sheet which is disposed so as to cover the water absorption material at a position of the display section inside the main body and has varying color tones between a water absorption state and a dry state.

2. The soil moisture indicator according to claim 1, wherein the water absorption material is formed of a cotton cloth from which oil is removed.

3. The soil moisture indicator according to claim 1 or 2, wherein the evaporation opening is formed on a side surface of the main body.

4. The soil moisture indicator according to any one of claims 1 to 3, wherein at least a part of the evaporation opening is provided on the other end side further than the display section.

5. The soil moisture indicator according to any one of claims 1 to 4, wherein the water absorption opening is formed on the side surface of the main body.

6. The soil moisture indicator according to any one of claims 1 to 5, wherein the main body is formed of a resin material.

## Amended claims

### Amended claims under Art. 19.1 PCT

1. A soil moisture indicator comprising:
a main body which is formed in a hollow shape by a material through which water does not pass, and has a water absorption opening disposed near one end in a longitudinal direction, an evaporation opening disposed near the other end, and a display section provided near the other end and formed to visualize the hollow inside;
a water absorption material which is filled on the inside of the main body at least from the water absorption opening to the display section; and
a water detection sheet which is disposed so as to cover the water absorptionmaterial at a position of the display section inside the main body and has varying color tones between a water absorption state and a dry state,
wherein the position and the magnitude of at least one of the water absorption opening and the evaporation opening, and a water absorption force of the water absorption material are set so that the water detection sheet enters the water absorption state when a pF value suitable for the plant to be grown is achieved.

2. The soil moisture indicator according to claim 1, wherein the number of the evaporation openings is set so that the water detection sheet enters the water absorption state when the pF value suitable for the plant to be grown is achieved.

3. The soil moisture indicator according to claim 1 or 2, wherein the pF value suitable for the plant to be grown is within a range of 2.1 to 2.6.

4. The soil moisture indicator according to claim 3, wherein the water absorption material is formed of a cotton cloth from which oil is removed.

5. The soil moisture indicator according to claim 3, wherein the water detection sheet has a hydrochromic ink.

6. The soil moisture indicator according to claim 4 or 5, wherein the water detection sheet is formed of a material having water retention capability inferior to the water absorption material.

7. The soil moisture indicator according to claim 4 or 5, wherein a preservative is contained in the water absorption material, and the position and the magnitude of at least one of the water absorption opening and the evaporation opening are determined so that the water detection sheet enters the water absorption state when the pF value suitable for the plant to be grown is achieved, by the water absorption force of the water absorption material containing the preservative.

Statement under Art. 19.1 PCT
In claim 1, it is clear that the position and the magnitude of at least one of the water absorption opening and the evaporation opening, and the water absorption force of the water absorption material are set so that the water detection sheet enters the water absorption state when the pF value suitable for the plant to be grown is achieved.
 In claim 2, it is clear that the number of the evaporation openings is set so that the water detection sheet enters the water absorption state when the pF value suitable for the plant to be grown is achieved.
 In claim 3, it is clear that the pF value suitable for the plant to be grown is within a range of 2.1 to 2.6.
 In claim 4, it is clear that the water absorption material is formed of a cotton cloth from which oil is removed.
 In claim 5, it is clear that the water detection sheet has a hydrochromic ink.
 In claim 6, it is clear that the water detection sheet is formed of a material having water retention capability inferior to the water absorption material.
 In claim 7, it is clear that a preservative is contained in the water absorption material, and the position and the magnitude of at least one of the water absorption opening and the evaporation opening are determined so that the water detection sheet enters the water absorption state when the pF value suitable for the plant to be grown is achieved, by the water absorption force of the water absorption material containing the preservative.

The present invention has the effect of being able to set so that the water detection sheet enters the water absorption state when the pF value suitable for the plant to be grown is achieved by the configurations described in each claim that is clarified as described above.
